# EUROPÄISCHE PATENTSCHRIFT

(11) **EP 0 650 594 B1**
(45) Veröffentlichungstag und Bekanntmachung des Hinweises auf die Patenterteilung: **12.03.1997**
(21) Anmeldenummer: 93914594.2
(22) Anmeldetag: 01.07.1993
(51) Int. Cl.: G01N 33/50, G01N 33/493, C01B 21/26, G01N 31/22

(54) **VERFAHREN ZUR BESTIMMUNG VON URINRESTMENGEN IN MEHRWEGBEHÄLTERN**
METHOD FOR DETERMINING THE AMOUNT OF RESIDUAL URINE IN REUSABLE BOTTLES AND CONTAINERS
PROCEDE DE DETECTION DE QUANTITES RESIDUELLES D'URINES DANS DES RECIPIENTS REUTILISABLES

(30) Priorität: 16.07.1992 DE 4223427
(43) Veröffentlichungstag der Anmeldung: 03.05.1995
(73) Patentinhaber: KHS Maschinen- und Anlagenbau Aktiengesellschaft, D-44143 Dortmund (DE)
(72) Erfinder: HEIDRICH, Karl, D-45355 Essen (DE)
(86) Internationale Anmeldenummer: DE9300586
(87) Internationale Veröffentlichungsnummer: WO9402848

(56) Entgegenhaltungen:
- US-A- 3 904 371
- US-A- 3 904 740

## Beschreibung

Die Erfindung betrifft ein Verfahren zur Bestimmung von Urinrestmengen in Mehrwegbehältern wie Flaschen in der Getränkeoder Lebensmittelindustrie.
Bislang ist für diesen Nachweis kein Verfahren bekannt, das eine sichere Identifikation des kontaminierten Gefäßes ermöglicht; dies gilt insbesondere für frische Urinrestmengen.

Aus der US-A-3 904 371 ist ein Verfahren zum Bearbeiten von Ammoniak in verschiedenen Schritten unter Einsatz einer möglichst niedrigen Temperatur bekannt.

Ferner beschreibt die US-A-3 904 740 ein Verfahren zur Herstellung von NO und NO₂.

Der Erfindung liegt deshalb die Aufgabe zugrunde, eine sichere Identifikation von mit Urin kontaminierten Gefäßen zu ermöglichen.

Diese Aufgabe wird gemäß der Erfindung dadurch gelöst, daß die aus dem Urin freigesetzten Ammoniakmengen in einem Konverter zu Stickstoffmonoxid gewandelt und anschließend mit Ozon in einer Chemilumineszenzreaktion nachgewiesen wird.

In dem erfindungsgemäßen Verfahren wird die Aufgabe dadurch gelöst, daß das im Urin entstehende Ammoniak zunächst in ein Hilfsgas (Stickstoffmonoxid) transferiert und der Nachweis in einem zweiten Schritt über die Chemilumineszenzreaktion des Stickoxids mit Ozon möglich wird. die Sekundärreaktion dient seit Jahren dem Nachweis von Stickoxiden in der Immissionsmeßtechnik. Entscheidend für die Verwendbarkeit des Verfahrens sind die geringe Nachweisgrenze (10 ppb), die selbst bei frischem Urin mit einem Ammoniakgehalt von etwa 3 ppm im Gasraum des Behälters eine sichere Identifikation erlaubt.
Die Umsetzung solch kleiner Gasmengen gelingt nur sicher, wenn katalytische oder Hochtemperaturkonvertoren oder Kombinationen aus beiden eingesetzt werden können. Für diese spezifische Applikation ist außerdem eine extrem kurze Meßdauer einzuplanen, da die Taktzeiten der Reinigungsmaschinen zwischen 140 und 200 msec variieren. Es ist deshalb vorteilhaft, den Konverter im Volumen zu begrenzen. Deshalb besteht der Konverter im Optimum aus einer Katalysatorkapillare, die gleichzeitig auch die Flußbegrenzung des Meßgases sichert und damit auch eine Voraussetzung schafft, den für die Chemilumineszenz wichtigen Unterdruck in der Meßzelle zu realisieren.

Entscheidend für die Umsetzung des Ammoniaks in NO ist die Temperatur des Konverters. Nur im Hochtemperaturkonverter ist es gewährleistet, daß das NO nicht bereits vor dem Erreichen der Meßzelle in NO₂ umgewandelt wird. Temperaturen über 600 °C sind mit Widerstandsheizungen nur noch schwer zu erzeugen. Deshalb wird die Konverterkapillare im Brennpunkt eines Parabolspiegels angeordnet. Die Beheizung erfolgt indirekt über einen zylindrischen Infrarotstrahler, dessen Energie auf die Kapillare fokussiert wird. Diese Einrichtung sichert so den Wärmetransfer auf das Meßgas und verhindert die vorzeitige Wandlung des NO's zu NO₂.

Zweckmäßig wird als Katalysatormaterial Platin, eine Platin-/ Iridiumverbindung, eine Platin-/Rhodiumverbindung auf einem Trägermaterial, insbesondere auf Silica- oder Alumina-Pellets oder auf einem Trägernetz aufgebracht.

Zur Beheizung des Katalysators wird die Energie eines Infrarotstrahlers mittels Parabolspiegels auf den Konverter fokussiert.

Der Konverter kann als Kapillare ausgebildet sein und die Doppelfunktion von Konverter und Strömungsbegrenzer erfüllen und dabei auf Grund des geringen Volumens die Spülzeit der Nachweiseinrichtung sowie die Anstiegszeit auf 140 - 200 msec begrenzen. Die Kapillare kann aus dem in Anspruch 6 genannten Katalysatormaterial gefertigt sein. Zur Kontrolle der Nullpunktstabilität kann eine Gasphasentitration mit Ozonh vorgeschaltet werden.

## Patentansprüche

1. Verfahren zum Nachweis von Urinrestmengen in Mehrwegbehältern wie Flaschen, **dadurch gekennzeichnet,** daß die aus dem Urin freigesetzten Ammoniakmengen in einem Konverter zu Stickstoffmonoxid gewandelt und anschließend mit Ozon in einer Chemilumineszenzreaktion nachgewiesen werden.

2. Verfahren nach Anspruch 1, **dadurch gekennzeichnet,** daß die Behälter abgesaugt und das Ammoniak/Luftgemisch in einem katalytischen Konverter in das Hilfsgas NO gewandelt wird.

3. Verfahren nach Anspruch 1, **dadurch gekennzeichnet,** daß das abgesaugte Luftgemisch in einem Hochtemperaturkonverter in das Hilfsgas NO gewandelt wird.

4. Verfahren nach den Ansprüchen 1 bis 3, **dadurch gekennzeichnet,** daß das Hilfsgas NO mit Ozon in einer Chemilumineszenzreaktion zu Stickstoffdioxid gewandelt wird.

5. Verfahren nach den Ansprüchen 1 bis 4, **dadurch gekennzeichnet,** daß das bei dem Chemilumineszenzprozeß erzeugte Licht zum Nachweis des Ammoniaks dient.

6. Verfahren nach Anspruch 2, **dadurch gekennzeichnet,** daß als Katalysatormaterial Platin, eine Platin-/Iridiumverbindung, eine Platin-/Rhodiumverbindung auf einem Trägermaterial, insbesondere auf Silica- oder Alumina-Pellets oder auf einem Trägernetz aufgebracht ist.

7. Verfahren nach den Ansprüchen 2 und 3, **dadurch gekennzeichnet,** daß zur Beheizung des Katalysators die Energie eines Infrarotstrahlers mittels Parabolspiegels auf den Konverter fokussiert wird.

8. Verfahren nach Anspruch 7, **dadurch gekennzeichnet,** daß der Konverter als Kapillare ausgelegt wird.

9. Verfahren nach den Ansprüchen 1 bis 8, **dadurch gekennzeichnet,** daß die Kapillare die Doppelfunktion von Konverter und Strömungsbegrenzer erfüllt und dabei auf Grund des geringen Volumens die Spülzeit der Nachweiseinrichtung und damit die Anstiegszeit auf 140 - 200 msec begrenzt.

10. Verfahren nach den Ansprüchen 6 bis 9, **dadurch gekennzeichnet,** daß die Kapillare aus dem in Anspruch 6 genannten Katalysatormaterial gefertigt ist.

11. Verfahren nach den Ansprüchen 1 bis 10, **dadurch gekennzeichnet,** daß zur Kontrolle der Nullpunktstabilität eine Gasphasentitration mit Ozon vorgeschaltet wird.

## Claims

1. Method for determining the amount of residual urine in reusable bottles and containers wherein the ammonia released from the urine is burned in a connverter to produce nitrogen monoxide, which is then reacted with ozone in a chemiluminescent reaction which then provides a measure of the ammonia present.

2. Method according to claim 1 wherein the air is exhausted from the containers and the ammonia/air mixture is converted into nitrogen monoxide (NO).

3. Method according to claim 1, wherein the air mixture exhaused from the containers is converted into nitrogen monoxide (NO) in a high-temperature converter.

4. Method according to the preceding claims 1 to 3, wherein the nitrogen monoxide (NO) is reacted with ozone in a chemiluminescent reaction to produce nitrogen dioxide.

5. Method according to the preceding claims 1 to 4, wherein the light produced in the chemiluminescent process serves the detection of ammonia.

6. Method according to claim 2, wherein the catalyst consists of platinum, a platinum-iridium compound, a platinum-rhodium compound on a carrier material, especially silica- or alumina pellets or gauze.

7. Method according to the preceding claims 2 and 3, wherein the energy of an infrared radiator is focussed on the converter with a reflector for heating the catalyst.

8. Method according to claim 7, wherein the converter may be designed as capillary tube.

9. Method according to the preceding claims 1 to 8, wherein the capillary tube has the double function of a converter and flowmeter and - due to its small volume - restricts the rinsing time of the analyzer and, by this, the testing time to 140 - 200 msec.

10. Method according to the preceding claims 6 to 9, wherein the capillary tube consists of a material as described under claim 6.

11. Method according to the preceding claims 1 to 10, wherein a gas phase titration with ozone can be interposed for the control of the original stability.

## Revendications

1. Procédé de détection de quantités résiduelles d'urine dans des récipients réutilisables tels que des bouteilles, caractérisé en ce que les quantités d'ammoniac libérées par l'urine sont transformées en monoxyde d'azote dans un convertisseur et sont ensuite mises en évidence avec de l'ozone, dans une réaction de chimioluminescence.

2. Procédé selon la revendication 1, caractérisé en ce que les récipients sont aspirés et en ce que le mélange ammoniac/air est transformé, dans un convertisseur catalytique, en gaz auxiliaire NO.

3. Procédé selon la revendication 1, caractérisé en ce que le mélange d'air aspiré est transformé, dans un convertisseur à haute température, en gaz auxiliaire NO.

4. Procédé selon les revendications 1 à 3, caractérisé en ce que le gaz auxiliaire NO, au moyen d'ozone, est transformé en dioxyde d'azote dans une réaction de chimioluminescence.

5. Procédé selon les revendications 1 à 4, caractérisé en ce que la lumière produite lors de la réaction de chimioluminescence sert à détecter l'ammoniac.

6. Procédé selon la revendication 2, caractérisé en ce qu'un catalyseur, constitué de platine, d'un mélange de platine et d'iridium, ou d'un mélange de platine et de rhodium, est appliqué sur un support, en particulier sur des boulettes de silice ou d'alumine, ou sur un maillage porteur.

7. Procédé selon les revendications 2 et 3, caractérisé en ce que, pour chauffer le catalyseur, l'énergie d'un rayonnement infrarouge est focalisée sur le convertisseur, par l'intermédiaire d'un miroir parabolique.

8. Procédé selon la revendication 7, caractérisé en ce que le convertisseur est conformé en tube capillaire.

9. Procédé selon les revendications 1 à 8, caractérisé en ce que le tube capillaire sert à la fois à la conversion et à limiter l'écoulement, limitant ainsi, du fait du faible volume, le temps de rinçage du dispositif de détection et, ainsi, le temps de montée, à 140-200 msec.

10. Procédé selon les revendications 6 à 9, caractérisé en ce que le tube capillaire est fabriqué dans le matériau catalyseur mentionné à la revendication 6.

11. Procédé selon les revendications 1 à 10, caractérisé en ce qu'afin de contrôler la stabilité du zéro, il est prévu, en amont, un titrage de phases gazeuses avec de l'ozone.
